# EUROPEAN PATENT SPECIFICATION

(11) **EP 3 378 449 B1**
(45) Date of publication and mention of the grant of the patent: **23.08.2023**
(21) Application number: 15908798.0
(22) Date of filing: 20.11.2015
(51) Int. Cl.: A61F 9/007

(54) **METHOD FOR PRODUCING PROBE FOR VITREOUS BODY OPERATION**
VERFAHREN ZUR HERSTELLUNG EINER SONDE FÜR GLASKÖRPEROPERATIONEN
PROCÉDÉ DE PRODUCTION DE SONDE POUR UNE OPÉRATION SUR LE CORPS VITRÉ

(43) Date of publication of application: 26.09.2018
(73) Proprietor: MANI, INC., Utsunomiya-shi, Tochigi 321-3231 (JP)
(72) Inventor: MURAKAMI, Etsuo, Utsunomiya-shi Tochigi 321-3231 (JP); OGANE, Kaoru, Utsunomiya-shi Tochigi 321-3231 (JP)
(74) Representative: Isarpatent
(86) International application number: PCT/JP2015/082663
(87) International publication number: WO 2017/085858

(56) References cited:
- EP-A1- 2 891 477
- WO-A1-2014/034506
- JP-A- H09 174 267
- JP-A- 2009 511 169
- US-A1- 2012 239 071
- US-A1- 2012 239 071

## Description

### Technical Field

The present invention relates to a method for manufacturing a vitreous body surgical probe used in ophthalmic surgery.

### Background Art

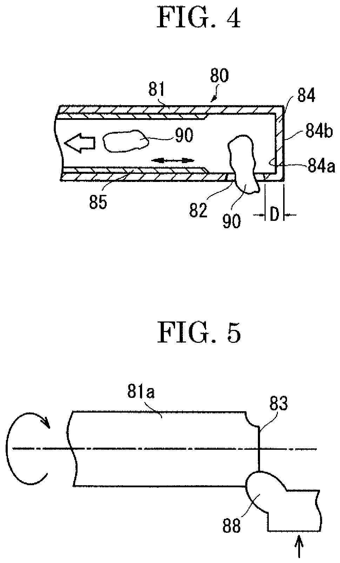

A vitreous body surgical probe used in ophthalmic surgery is used for cutting and removing from an eyeball a jelly-like vitreous body and/or a proliferative membrane on the retina generated through denaturation of the vitreous body. FIG. 4 shows a cross-section of a vitreous body surgical probe.

A vitreous body surgical probe 80 includes a probe main body 81 having a form with a sealed, pipe end part 84, and a cutting member 85, which is slidable in the axial direction on the inner surface of the probe main body 81 with continuous contact on that surface. An opening 82 is provided in a side near the end of the probe main body 81, and vitreous bodies etc. 90 are sucked in through the opening. At this time, the vitreous bodies 90 are cut when the cutting member 85 slides and the end of the cutting member 85 passes by the opening 82, and the vitreous bodies etc. 90 that are cut into small pieces are sucked in at the back side of the probe (left side of FIG. 4) and collected.

It is preferable that the vitreous body surgical probe 80 has a short distance D between the end part 84 and the opening 82. This is because since the vitreous bodies etc. 90 are either near the retina or are floating in the vicinity of the retina, provision of the opening 82 as close to the retina as possible is required. Moreover, an end outer surface 84b is preferably a flat surface without any protrusions so that the probe main body 81 does not touch and damage the retina.

It is preferable that an end inner surface 84a is also a flat surface. This is because if the end inner surface 84a is not a flat surface, it is difficult to bring the end of the cutting member 85 near the end part 84 of the probe main body 81 when making the cutting member 85 slide, and therefore the distance D from the end part 84 to the opening 82 needs to be made long.

As a method for forming such an end part 84 of the probe main body 81, a deformation processing method of squeezing an end part of a pipe, so as to form the probe main body 81 is disclosed in Patent Document 1. FIG. 5 is a diagram describing a conventional deformation processing method for an end surface. Note that a pipe to undergo deformation processing at the end part is hereafter referred to as a deformation-processed pipe.

Patent Document 1 discloses a method of shifting a spherical protrusion 88 along the radius of the deformation-processed pipe 81a while rotating a deformation-processed pipe 81a around the principal axis and pressing the spherical protrusion 88 against a pipe end part 83. As a result, an end portion of the deformation-processed pipe 81a is gradually deformed inwardly, ultimately forming the end part. Such a deformation processing method through squeezing has a drawback that while processing is easy and the end outer surface may thus be formed nearly flat, burrs easily form on the end inner surface.

### Prior Art Documents

### Patent Documents

[Patent Document 1] JP 2009-511169, [Patent Document 2] US2012239071

### DISCLOSURE OF INVENTION

### Problem to be Solved by the Invention

In light of the problem, the present invention aims to provide a method for manufacturing a vitreous body surgical probe that is easily manufactured and has a flat end outer surface and a flat end inner surface.

### Means of Solving the Problem

The present invention is set out in the appended claims. Any embodiments, examples or aspects according to the present disclosure which do not fall within the scope of said claims are provided for illustrative purposes only and do not form part of the present invention.

### BRIEF DESCRIPTION OF DRAWINGS

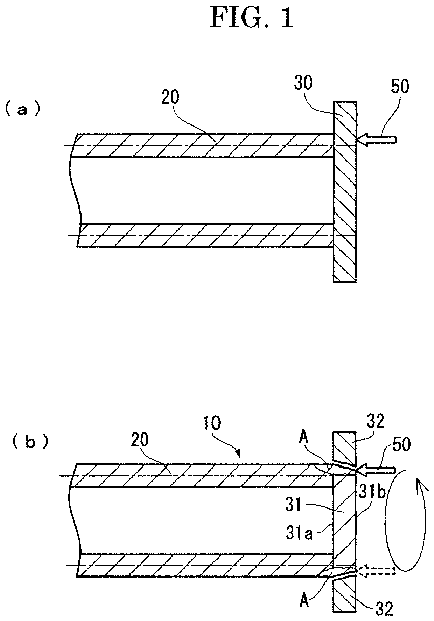

FIG. 1 shows cross-sections describing a process of cutting and welding a steel plate simultaneously so as to form an end of a vitreous body surgical probe, wherein FIG. 1(a) illustrates the state when starting to irradiate an energy beam, and FIG. 1(b) illustrates the state after having irradiated the energy beam;

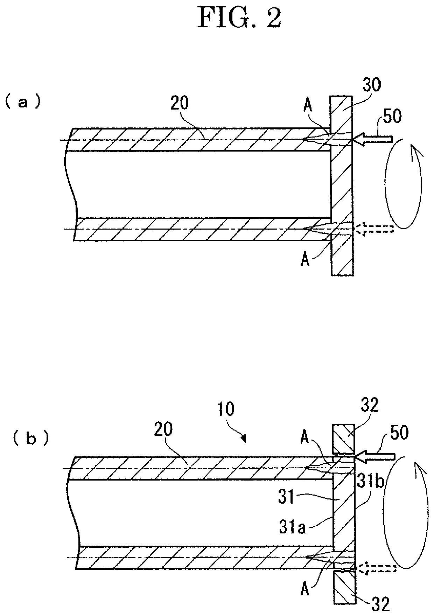

FIG. 2 shows cross-sections describing a process of forming the end of the vitreous body surgical probe, wherein FIG. 2(a) illustrates the state when irradiating an energy beam so as to weld together a steel plate and a pipe, and FIG. 2(b) illustrates the state when irradiating an energy beam so as to cut the steel plate;

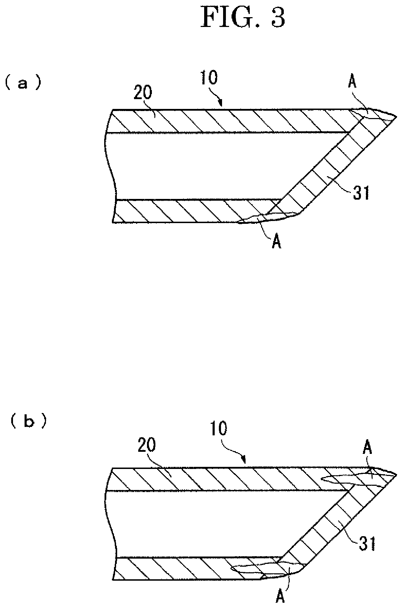

FIG. 3 shows cross-sections illustrating a case where the end of the vitreous body surgical probe is a slanted surface, wherein FIG. 3(a) illustrates the case where cutting and welding using an energy beam are carried out simultaneously, and FIG. 3(b) illustrates the case where cutting is carried out after having welded;
FIG. 4 shows a cross-section of the vitreous body surgical probe; and
FIG. 5 is a diagram describing a conventional deformation processing method for an end surface.

### DESCRIPTION

An example according to the present description is described below with reference to accompanying drawings.

FIG. 1 shows cross-sections describing a process of cutting and welding a steel plate simultaneously so as to form an end of a vitreous body surgical probe, wherein FIG. 1(a) illustrates the state when starting to irradiate an energy beam, and FIG. 1(b) illustrates the state after having irradiated the energy beam. The main body of a vitreous body surgical probe 10 has a structure where an end surface of a pipe 20 is closed. A process of closing the end surface of the pipe 20 is described below.

To begin with, a steel plate 30 to be formed into the end part of the vitreous body surgical probe 10 is brought into contact with an end surface of the steel pipe 20. An energy beam 50 is then irradiated from the steel plate 30 side. The irradiation point of the energy beam 50 at this time is shifted along the outer circumference of the end surface of the pipe 20.

Here, an appropriate intensity of the energy beam 50 value makes it possible to cut the steel plate 30 into an end part 31 and fragments 32, and thereby welding the end part 31 to the outer circumference of the end surface of the pipe 20 simultaneously. Note that the remaining fragments 32 resulting from cutting the end part 31 of the steel plate 30 are cut off.

As a result of the energy beam 50 irradiated along the outer circumference of the pipe 20, weld-affected zones A range to the outer rim of the pipe 20 from approximately the center of the thickness of the pipe 20. Note that the inner circumference side of the pipe 20 is not welded to the end part 31.

An appropriate intensity of the energy beam 50 should be set. If the intensity is too strong here, the weld-affected zones A are too wide, and a problem that keeping an end inner surface 31a and an end outer surface 31b of the end part 31 flat is impossible occurs.

However, since the vitreous body surgical probe 10 have a structure where a strong force is not applied to the weld-affected zones A, and the steel plate 30 itself is a relatively thin steel plate, it is possible to cut and weld the steel plate 30 simultaneously even without making the intensity of the energy beam 50 very strong. Therefore, as long as an appropriate intensity of the energy beam 50 is set, the energy beam 50 needs to be irradiated only one time, thereby allowing substantial reduction in manufacturing operations.

FIG. 2 shows cross-sections describing a process of forming the end of the vitreous body surgical probe, wherein FIG. 2(a) illustrates the state when irradiating an energy beam so as to weld together a steel plate and a pipe, and FIG. 2(b) illustrates the state when irradiating an energy beam so as to cut the steel plate. A manufacturing process of welding the pipe 20 to the steel plate 30 and then cutting the steel plate 30 to form the end part 31 is described here.

To begin with, the steel plate 30 to be formed into the end part 31 of the vitreous body surgical probe 10 is brought into contact with an end surface of the steel pipe 20. The energy beam 50 is then irradiated from the steel plate 30 side. The irradiation point of the energy beam 50 at this time moves along the circumference of the pipe 20 near the center of the thickness of the pipe 20. By irradiation with this energy beam 50, the pipe 20 and the steel plate 30 are welded together.

Next, irradiation of the energy beam 50 is temporarily stopped, and irradiation is then restarted from the steel plate 30 side along the outer circumference of the pipe 20. Through this operation, the steel plate 30 is cut along the outer circumference of the pipe 20, and is divided into the cut off fragments 32 and the end part 31 that is joined to the pipe 20.

In this process, even though the energy beam 50 is irradiated twice, there is a merit that welding is surely performed. Moreover, if the same energy beam 50 is irradiated twice, the intensity of the energy beam 50 does not need to be changed, and only an operation of slightly shifting the irradiation point is added. Therefore, a reliable product may be supplied without much increase in workload.

There are cases where a slanted end surface is preferable so as to make a structure allowing the end of the vitreous body surgical probe to be brought as close to the retina as possible. FIG. 3 shows cross-sections illustrating a case where the end of the vitreous body surgical probe is a slanted surface, wherein FIG. 3(a) illustrates the case where cutting and welding through irradiation of an energy beam are carried out simultaneously, and FIG. 3(b) illustrates the case where cutting is carried out after having welded.

In order to make the end part 31 of the vitreous body surgical probe 10 a slanted surface, an end surface of the steel pipe 20 needs to have a slanted cut form. That is, the end surface should have an elliptic form. A steel plate is then brought into contact with the end surface of the pipe 20, and an energy beam is irradiated from the steel plate side. Here, irradiation of an energy beam includes, as in the case where the end is at a right angle with the pipe, simultaneous cutting and welding of the steel plate through one irradiation, and cutting of the steel plate after the steel plate has been welded to the pipe 20, thereby forming the end part 31. That is, even the end part 31 with a slanted surface may be manufactured very easily.

Moreover, such a structure where the end part 31 of the vitreous body surgical probe 10 has a slanted surface cannot be manufactured by a conventional method of squeezing the end part; however, according to the present description, it can be easily manufactured. Furthermore, both the inner and outer surfaces of the end part 31 may be finished as flat surfaces, thereby having good workability.

In this manner, according to the method of manufacturing the vitreous body surgical probe according to the present description, the end part of the probe main body may be easily manufactured. The case where welding and cutting are carried out simultaneously (FIG. 3(a)) has a merit that irradiation of the energy beam is only required one time since the range of the weld-affected zones A is small, and the case where welding and cutting are carried out separately (FIG. 3(b)) has a merit that through use of the same energy beam for welding and cutting, welding may be carried out surely without much increase in workload.

### Explanation of References

10: Vitreous body surgical probe
20: Pipe
30: Steel plate
31: End part
31a: End inner surface
31b: End outer surface
32: Fragment
50: Energy beam
A: Weld-affected zone

## Claims

1. A method for manufacturing a vitreous body surgical probe (10), comprising the steps of:
bringing a flat steel plate (30) in contact with an end surface of a steel pipe (20);
irradiating an energy beam (50) from the steel plate (30) side and moving the point of the energy beam (50) along the circumference of the pipe (20) end surface at the center of the thickness of the pipe (20) so as to weld together the steel plate (30) and the center of the thickness of the pipe (20); and
temporarily stopping irradiation of the energy beam (50), and restarting irradiation of the same energy beam (50) from the steel plate (30) side along the outer circumference of the pipe (20) end surface, so as to cut the steel plate (30) along the outer circumference of the pipe (20).

2. The method for manufacturing a vitreous body surgical probe (10) of Claim 1, wherein the end surface of the pipe is cut at a slant.

## Patentansprüche

1. Verfahren zur Herstellung einer chirurgischen Glaskörper-Sonde (10), welches die folgenden Schritte umfasst:
in Kontakt bringen einer flachen Stahlplatte (30) mit einer Endfläche eines Stahlrohrs (20);
Bestrahlen der Seite der Stahlplatte (30) entlang des Umfangs der Endfläche des Rohrs (20), in der Mitte der Dicke des Rohrs (20), mit einem Energiestrahl (50), um die Stahlplatte (30) und das Rohr in der Mitte der Dicke des Rohrs (20) zusammenzuschweißen; und
vorübergehendes Unterbrechen der Bestrahlung durch den Energiestrahl (50) und erneutes Beginnen der Bestrahlung der Seite der Stahlplatte (30) mit demselben Energiestrahl (50) entlang des Außenumfangs der Endfläche des Rohrs (20), um die Stahlplatte (30) entlang des Außenumfangs des Rohrs (20) abzuschneiden.

2. Verfahren zur Herstellung einer chirurgischen Glaskörper-Sonde (10) nach Anspruch 1, wobei die Endfläche des Rohrs schräg abgeschnitten wird.

## Revendications

1. Procédé de fabrication d'une sonde chirurgicale à corps vitré (10), comprenant les étapes suivantes :
Mettre une plaque d'acier plate (30) en contact avec une surface d'extrémité d'un tube d'acier (20);
irradiation du côté de la plaque d'acier (30) le long de la circonférence de la surface d'extrémité du tube (20), au milieu de l'épaisseur du tube (20), avec un faisceau d'énergie (50) pour souder ensemble la plaque d'acier (30) et le tube au milieu de l'épaisseur du tube (20); et
interrompre temporairement l'irradiation par le faisceau d'énergie (50) et recommencer à irradier le côté de la plaque d'acier (30) avec le même faisceau d'énergie (50) le long de la circonférence extérieure de la surface d'extrémité du tube (20) pour couper la plaque d'acier (30) le long de la circonférence extérieure du tube (20).

2. Procédé de fabrication d'une sonde chirurgicale à corps vitré (10) selon la revendication 1, dans lequel la surface d'extrémité du tube est coupée en biais.
